# FASCICULE DE BREVET EUROPEEN

(11) **EP 1 274 892 B1**
(45) Date de publication et mention de la délivrance du brevet: **04.11.2009**
(21) Numéro de dépôt: 01928031.2
(22) Date de dépôt: 20.04.2001
(51) Int. Cl.: D04B 21/04, A61F 2/00

(54) **TRICOT PROTHETIQUE AGRIPPANT ET IMPLANT DE RENFORT POUR LE TRAITEMENT DES DEFICITS PARIETAUX**
HAFTENDES PROTHESENGEWIRK UND VERSTÄRKUNGSIMPLANTAT ZUR BEHANDLUNG VON ZELLWANDDEFIZITEN
ADHERING PROSTHETIC KNITTING FABRIC AND REINFORCEMENT IMPLANT FOR TREATING PARIETAL DEFICIENCIES

(30) Priorité: 20.04.2000 FR 0005124
(43) Date de publication de la demande: 15.01.2003
(73) Titulaire: SOFRADIM PRODUCTION, 01600 Trévoux (FR)
(72) Inventeur: ORY, François-Régis, F-69270 Fontaines Saint Martin (FR); THERIN, Michel, F-69002 Lyon (FR); MENEGHIN, Alfredo, F-69480 Anse (FR)
(74) Mandataire: Guerre, Dominique
(86) Numéro de dépôt international: PCT/FR2001/001234
(87) Numéro de publication internationale: WO 2001/081667

(56) Documents cités:
- EP-A- 0 276 890
- EP-A- 0 827 724
- WO-A-99/66860
- DE-A- 19 832 634
- FR-A- 2 766 698
- US-A- 4 338 800
- US-A- 5 254 133

## Description

L'invention est relative à un tricot prothétique agrippant, à un procédé de fabrication de ce tricot prothétique et à un implant de renfort réalisé avec ce tricot prothétique, permettant le traitement des déficits pariétaux.

Dans le traitement des insuffisances pariétales (hernies et éventrations principalement), le renfort a pour vocation d'apporter un soutien mécanique permanent à la reconstruction chirurgicale. Le renfort est d'autant plus efficace et sa tolérance locale d'autant meilleure que son intégration tissulaire est intime et précoce. Pour être intime et précoce sans formation de coque fibreuse périphérique, les macro porosités de l'implant doivent le plus largement possible être ouvertes sur l'extérieur et l'élasticité du renfort doit lui permettre de suivre les déformations physiologiques de la paroi. Les limites sont fixées par la résistance mécanique du textile, qui doit rester supérieure à 10 décaNewtons en test normalisé type ISO5081, par la maniabilité par le chirurgien et par l'impossibilité de récidive de hernie par les pores du tissu qui doivent au maximum avoir 7 à 10 millimètres de diamètre, par exemple.

La notion de renfort tissulaire de paroi abdominale sous la forme de textile tricoté est connue depuis des décennies dans la littérature scientifique. Un certain nombre de solutions techniques ont été décrites, en particulier dans les documents suivants : US 5 569 273, WO 96/03091, EP 0 797 962, FR 2 766 698. Elles ont toutes en commun le tricotage de mono ou multifilament en polypropylène ou en polyester.

De façon connue, de tels renforts tissulaires, dénommés aussi tissus ou tricots prothétiques, doivent satisfaire à de nombreuses contraintes, et en particulier présenter une résistance mécanique dans au moins deux directions perpendiculaires, être biocompatibles, souples et conformables tout en présentant une certaine élasticité, dans au moins une direction, être poreux et transparents, être suturables et redécoupables tout en étant indéchirables et indémaillables, et enfin, être stérilisables et pérennes. En général, ces renforts sont tricotés et composés de plusieurs nappes de fils entremêlés formant une structure dite tridimensionnelle, en ce qu'elle présente à plat une certaine épaisseur.

Des mises en forme spécifique de ces textiles de façon à se conformer, d'une part, à l'anatomie de la région inguinale et, d'autre part, à la technique chirurgicale employée sont également connues depuis de nombreuses années, aussi bien pour les voles d'abord postérieures (EP 0 836 838, WO 95/07666, WO 96/41588) que pour les voies d'abord antérieures (US 5 356 432, EP 0 827 724). Si d'une façon générale, les renforts conçus pour les voies d'abord postérieures sont de grandes dimensions et ne nécessitent que peu de points de fixation, les renforts conçus pour les voies d'abord antérieures sont de plus petites dimensions, sont fendus (à l'avance ou extemporanément) pour entourer le cordon spermatique et nécessitent donc une fixation relativement extensive, d'une part, pour fermer la fente sur elle-même, d'autre part, pour s'opposer aux forces intra-abdominales d'extrusion, et, enfin, pour garantir une intégration rapide aux tissus périphériques.

Le document EP 0 276 890 A2 décrit un textile dense a six nappes de fils, comprenant des picots, pour fermeture par contact sur un autre textile.

Le document DE 198 32 634 A1 décrit un implant qui comprend un volet se refermant sur ledit implant grâce à un système de velcro.

Le document US 5,254,133 décrit un inplant formé de trois éléments reliés entre eux par un système de velcro.

L'invention concerne plus spécialement tes implants de renfort comprenant deux éléments solidarisés par couture ou soudure et formant deux volets se superposant, au moins localement. L'invention a pour objet de fournir un tricot agrippant satisfaisant aux contraintes spécifiques des tricots prothétiques, rappelées ci-dessus, mais apportant pour tout implant de renfort réalisé avec ledit tricot, différents avantages, à savoir suppression possible de toute opération complémentaire de liaison entre les deux éléments, simplicité d'utilisation, rapidité et sécurisation de la mise en place pour le praticien, et efficacité de la réparation fonctionnelle.

La présente invention propose un tricot prothétique à usage médical ou chirurgical, dont la structure est réalisée en fil monofilament et multifilament, bio-compatible et partiellement biorésorbable.

Selon l'invention, ce tricot prothétique comprend au moins trois nappes, dont une nappe en monofil thermofusible formant, sur une face du tricot, des poils à picot saillant perpendiculairement à ladite nappe, c'est-à-dire des poils présentant chacun un corps sensiblement rectiligne et, du côté de l'extrémité libre de ce corps, une tète de largeur supérieure à celle de ce corps.

Ce tricot est en particulier susceptible d'être obtenu en utilisant un monofil thermo-fusible pour constituer ladite nappe en monofil, en formant, au niveau de cette nappe, des mailles extérieures en bouclettes, puis en réalisant la fusion partielle dudit monofil.

La structure textile du tricot comporte ou définit sur ses deux faces, dont celle comprenant les poils à picot, des pores ouverts, ayant par exemple un diamètre compris entre 1 et 3 mm.

Le monofil formant les poils à picot est en matériau bio-résorbable thermofusible, choisi dans le groupe consistant en les polymères de p-dioxanone, les polyglycolides, les polyorthoesters, les polymères de triméthylène carbonate, les stéréocopolymères de l'acide L et D tactique, les homopolymères de l'acide L lactique, les copolymères de l'acide lactique et d'un comonomère compatible, tels que les dérivés d'alpha-hydroxy acides.

La longueur des poils à picot est définie pour pénétrer et s'accrocher dans la structure textile du tricot, de manière limitée, c'est-à-dire sans en dépasser de l'autre face, par exemple lorsque le côté agrippant d'un tricot selon l'invention, comportant lesdits poils à picot est appliqué contre une face non agrippante, ne comportant pas lesdits poils, du même tricot ou d'un tricot différent.

Préférentiellement, le mono-fil formant les poils à picot a un diamètre supérieur à 0,10 mm, et/ou chaque poil à picot a une longueur comprise entre 1 et 2 mm, et/ou la densité des poils à picot est comprise entre 50 et 90 poils au centimètre carré.

A titre d'exemple, cette structure comporte plusieurs nappes de fils entremêlés formant ensemble une structure tridimensionnelle. Dans ce dernier cas, la structure tridimensionnelle est composée, par exemple de trois nappes, à savoir :
- une nappe intermédiaire en fil distribué pour former un jour en quinconce entre les colonnes de mailles,
- une nappe avant en fils distribués pour former une chaînette, et
- une nappe arrière en mono-fil, posée en trame partielle sous la chaînette et « jetée sur » l'aiguille ne faisant pas chaînette, cette nappe comportant les poils à picot.

Lorsqu'un tricot agrippant selon l'invention est appliqué, poils à picot en avant, sur un tricot prothétique sans poil, les poils à picot s'engagent dans les mailles et entre les fils multiflament du tricot du renfort et assurent le verrouillage du tricot agrippant sur l'autre tricot. Ce verrouillage, efficace même en milieu liquide, est suffisant pour sécuriser la fermeture d'une fente, s'il en existe une, et offrir une résistance mécanique aux sollicitations tangentielles, tout en permettant le décrochage du tricot agrippant pour ajuster sa position par rapport à l'élément sous jacent.

La présente demande décrit également un procédé de fabrication de ce tricot agrippant défini précédemment à titre d'exemple, et comprenant trois nappes, respectivement intermédiaire, avant, et arrière.

Ce procédé comprend :
(i) une phase de fabrication d'un tricot à bouclette sur un métier à mailles jetées à au moins trois nappes de fils et autant de barres à passettes, à savoir :
- une barre arrière enfilée, une passette pleine, une passette vide, par le monofil formant les poils à picot,
- une barre intermédiaire enfilée, une passette pleine, trois passettes vides, formant un fond léger ajouré et stable en largeur,
- une barre avant enfilée, une passette pleine, une passette vide, ces trois barres travaillant selon le barème suivant :

| | Barre arrière | Barre intermédiaire | Barre avant | |
|---|---|---|---|---|
| Chaîne→ | I | II | III | |
| | Barre avant | Barre intermédiaire | Barre arrière | |
| Rachel→ | I | II | III | |
| | 7 | 3 | 1 | |
| | 7 | 2 | 0 | |
| | --- | --- | --- | |
| | 3 | 4 | 0 | |
| | 4 | 5 | 1 | |
| | --- | --- | --- | |
| | 0 | 1 | | |
| | 0 | 0 | | |
| | --- | --- | | |
| | 4 | 2 | | |
| | 3 | 3 | | |
| | | --- | | |
| | | 1 | | |
| | | 0 | | |
| | | --- | | |
| | | 4 | | |
| | | 5 | | |

(ii) une phase de thermofixation du tricot à bouclettes, et
(iii) une phase de transformation des bouclettes en poils à picot par plaquage du tricot en défilement sur un cylindre porté à une température entraînant la fusion des bouclettes et la formation des picots.

L'invention concerne également toute utilisation d'un tricot tel que défini précédemment, pour l'obtention d'un objet prothétique, à usage médical ou chirurgical, par exemple un implant prothétique de renfort tissulaire.

L'invention concerne donc également un implant prothétique de renfort tissulaire, dont la caractéristique essentielle est qu'il est constitué au moins en partie par un tricot selon l'invention.

A titre d'exemple, un tel implant prothétique comprend une partie ou élément agrippant, par exemple un rabat, dont l'une des faces, dite agrippante, comprend des poils à picot, agencés et/ou disposés pour pénétrer de manière limitée et s'accrocher dans l'épaisseur de la structure textile d'une autre partie ou autre élément, par exemple non agrippant, pouvant appartenir au même implant ou à un autre objet ou partie prothétique, et ceci lorsque la face agrippante de l'élément agrippant, c'est-à-dire celui comportant les poils à picot, est disposé contre une face non agrippante, par conséquent sans poil à picot, dudit autre élément.

Préférentiellement, la totalité de l'élément agrippant est réalisé en un tricot selon l'invention.

Préférentiellement, l'élément agrippant est lié au reste de l'implant, par une zone de liaison, par exemple par couture ou soudure.

Préférentiellement, et comme décrit ci-après, l'élément agrippant est ledit autre élément, non agrippant, appartiennent au même implant, et sont par exemple respectivement deux volets se superposant localement. Dans ce dernier cas, les deux éléments, agrippant et non agrippant, sont agencés et/ou disposés l'un par rapport à l'autre, en sorte que dans leur position agrippée, les poils à picot demeurent à l'intérieur du périmètre ou du contour de l'implant, de façon à ce que les poils à picot ne puissent ultérieurement venir en contact avec les tissus biologiques environnants.

Dans l'implant de renfort selon l'invention, l'un des éléments est constitué par un tricot prothétique composé d'au moins trois nappes dont l'une est réalisée en monofil formant des poils à picot saillant d'une face en direction de l'autre élément et sur une longueur inférieure à l'épaisseur de la structure tricotée de cet autre élément, cet élément à poils à picot étant lié à l'autre élément en tricot prothétique, sans poil à picot, par une zone de liaison, par couture ou soudure, disposé dans la zone de superposition des deux éléments.

Grâce à cet aménagement, les poils à picot du tricot agrippant son localisés dans la zone de superposition des deux éléments de l'implant et ne peuvent, en aucun cas, irriter les tissus biologiques environnants.

D'autres caractéristiques et avantages ressortiront de la description qui suit en référence au dessin schématique annexé représentant, à titre d'exemples, une forme d'exécution du tricot agrippant et plusieurs formes d'exécution des implants le mettant en oeuvre.
Figure 1 est un graphique représentant à titre d'exemple l'armure des trois nappes composant le tricot agrippant,
Figure 2 est une vue schématique de côté d'une forme d'exécution du dispositif assurant la formation des poils à picot,
Figure 3 est une vue schématique de côté illustrant, de façon non conventionnelle, l'interpénétration des structure, respectivement, du tricot agrippant et du tricot prothétique dans lequel il s'agrippe,
Figures 4 à 6 sont des vues en perspective représentant trois formes d'exécution de renforts pariétaux, avec leur volet agrippant écarté pour montrer la structure et la forme de l'implant,
Figures 7 à 9 sont des vues en plan par-dessus des implants de figures 3 à 5, volets rabattus,
Figures 10 à 14 sont des vues de différentes armures qu'il est possible d'utiliser de manière alternative pour l'obtention du tricot agrippant selon l'invention.

Le tricot agrippant selon l'invention est réalisé sur un métier à manies jetées, de type chaîne ou rachel, avec au moins trois nappes ou chaînes de fil et autant de barres à passettes.

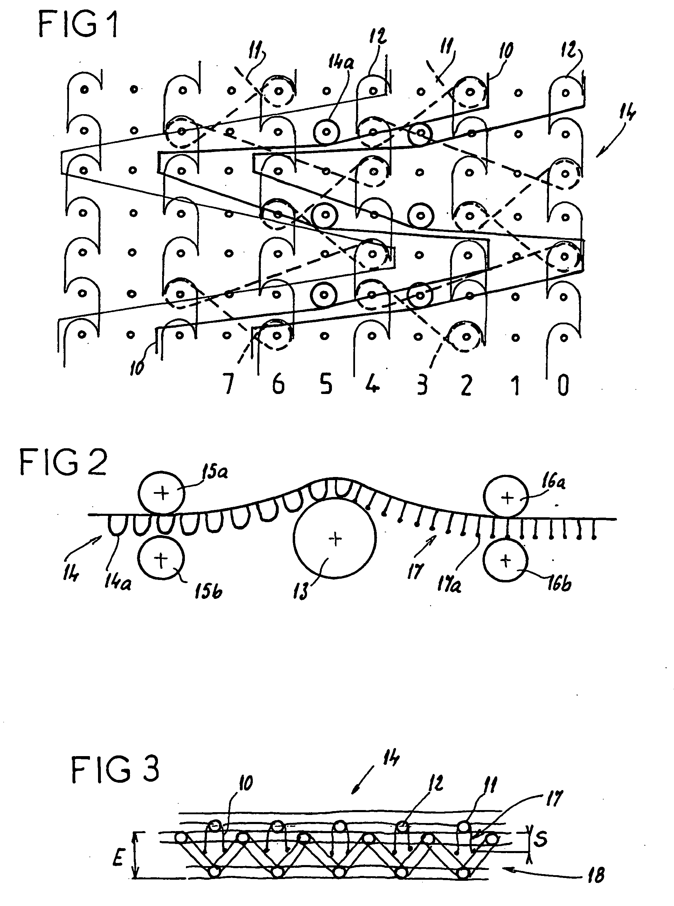

La barre de fond ou barre arrière est enfilée, une passette pleine, une passette vide, en monofil de polymère biocompatible et thermofusible, ayant un diamètre supérieur à 0,10 millimètres. En pratique ce diamètre est compris entre 0,14 et 0,18 millimètres et est de l'ordre de 0,15 millimètres. Ce fil est représenté par la référence 10 et en trait fort, à la figure 1.

La barre intermédiaire est enfilée, une passette pleine, trois passettes vides, en polyester multifilament, mais elle peut aussi être enfilée en polyester monofilament ou en polypropylène mono ou multifilament. Ce fil est représenté en trait moyen et par la référence 11 à la figure 1. La barre intermédiaire travaille de façon à obtenir un jour en quinconce entre les colonnes de mailles.

Enfin, la barre avant est enfilée une passette pleine, une passette vide et travaille en armure chaînette avec un fil multi ou monofilament en polyester ou polypropylène et, par exemple, en fil polyester multifilament. Ce fil est représenté en trait fin, avec la référence 12 à la figure 1. La chaînette emprisonne le monofil 10 et maintient le tricot en long, tout en contribuant à la réalisation du tricot avec la nappe intermédiaire formée par le fil 11. Les différents fils sont travaillés suivant le barème indiqué ci-après :

| | | | | |
|---|---|---|---|---|
| | Barre arrière | Barre intermédiaire | Barre avant | |
| Chaîne→ | I | II | III | |
| | Barre avant | Barre intermédiaire | Barre arrière | |
| Rachel→ | I | II | III | |
| | 7 | 3 | 1 | |
| | 7 | 2 | 0 | |
| | --- | --- | --- | |
| | 3 | 4 | 0 | |
| | 4 | 5 | 1 | |
| | --- | --- | --- | |
| | 0 | 1 | | |
| | 0 | 0 | | |
| | --- | --- | | |
| | 4 | 2 | | |
| | 3 | 3 | | |
| | | --- | | |
| | | 1 | | |
| | | 0 | | |
| | | --- | | |
| | | 4 | | |
| | | 5 | | |

La barre arrière pose le fil en trame partielle sous la chaînette et en « jetée sur », sur l'aiguille ne faisant pas chaînette. De ce fait, à la rangée suivante, l'aiguille ne faisant pas chaînette n'étant pas alimentée, laisse échapper la maille en monofil qui forme une bouclette 14a saillant de la face avant du tricot.

L'enfilage en passette pleine, trois passettes vides dans la barre intermédiaire, associé au déplacement, permet de réaliser un fond léger, stable en largeur, et ajouré permettant une bonne intégration tissulaire.

Le tricot 14, ainsi obtenu, est pourvu de bouclettes 14a (figure 2) perpendiculaires à l'une de ses faces et dont la rigidité et le maintien à angle droit sont obtenus par la rigidité ou nerf du monofil employé. Cette rigidité est nécessaire pour la formation ultérieure des poils à picot assurant la fonction agrippante.

Le monofil formant les bouclettes et ultérieurement les poils à picot est en matériau bio-résorbable thermofusible, choisi dans le groupe consistant en les polymères de p-dioxanone, les polyglycolides, les polyorthoesters, les polymères de trimèthylène carbonate, les stéréocopolymères de l'acide L et D lactique, les homopolymères de l'acide L lactique, les copolymères de l'acide lactique et d'un comonomère compatible, tels que les dérivés d'alpha-hydroxy acides.

En sortie de métier, le tricot 14 est soumis à une opération de thermofixation qui le stabilise en long et en large, puis il est soumis à une phase de formation des poils à picot consistant, comme montré à la figure 2, à le faire passer sur un cylindre 13 contenant une résistance électrique de chauffage. Le tricot 14 est plaqué sur le cylindre 13 par deux paires de rouleaux, respectivement amont 15a, 15b, et aval 16a, 16b déplaçables verticalement pour régler cette force de plaquage.

Ce réglage, de même que celui de la température de la résistance disposée dans le cylindre 13 et de la vitesse de défilement de la nappe 14 sur le cylindre, permettent de fondre la tête ou boucle de chacune des bouclettes 14a, de sorte que chaque bouclette 14a forme deux poils à picot 17.

Chaque poils à picot 17 présente ainsi un corps sensiblement rectiligne, saillant perpendiculairement à la nappe en monofil 10, et, du côté de l'extrémité libre de ce corps, une tête 17a de largeur supérieure à celle de ce corps. Cette tête 17a a une forme généralement sphéroïdale ou une forme de champignon.

La longueur S des poils 17, mesurée depuis la face dont ils font saillie perpendiculairement jusqu'au sommet de la tête 17a, est déterminée de manière qu'elle soit inférieure à l'épaisseur E de la structure tricotée 18 dans laquelle ils doivent pénétrer et s'accrocher, et est comprise entre 1 et 2 millimètres.

La structure tricotée 18 avec laquelle collabore le tricot agrippant 14, est un tissu prothétique satisfaisant aux contraintes rappelées dans le préambule, et en particulier, est un tricot tridimensionnel ajouré avec deux faces poreuses reliées par des fils de liaison. Il est, par exemple, réalisé par le tricot défini dans le brevet français n° 2 766 698.

Lorsque le tricot agrippant 14 est engagé, avec les poils à picot 17 en avant, en direction du tricot prothétique 18, les poils à picot 17 s'insèrent dans les mailles du tricot 18, et s'accrochent entre les filaments de ces mailles, sans pour autant dépasser du tricot 18, ce qui induirait le risque d'irriter les tissus biologiques environnants.

La densité des poils à picot 17 dépend de la jauge utilisée et du nerf et de la rigidité du monofil 10. Elle est comprise entre 50 et 90 poils au cm2. Cette densité est suffisante pour assurer l'agrippage tout en permettant le décrochage des deux tricots 14 et 18.

Un tel tricot est intéressant par exemple pour la fabrication d'implants de renfort devant être rabattus ou sécurisés sur eux-mêmes, et en particulier, d'implants pariétaux.

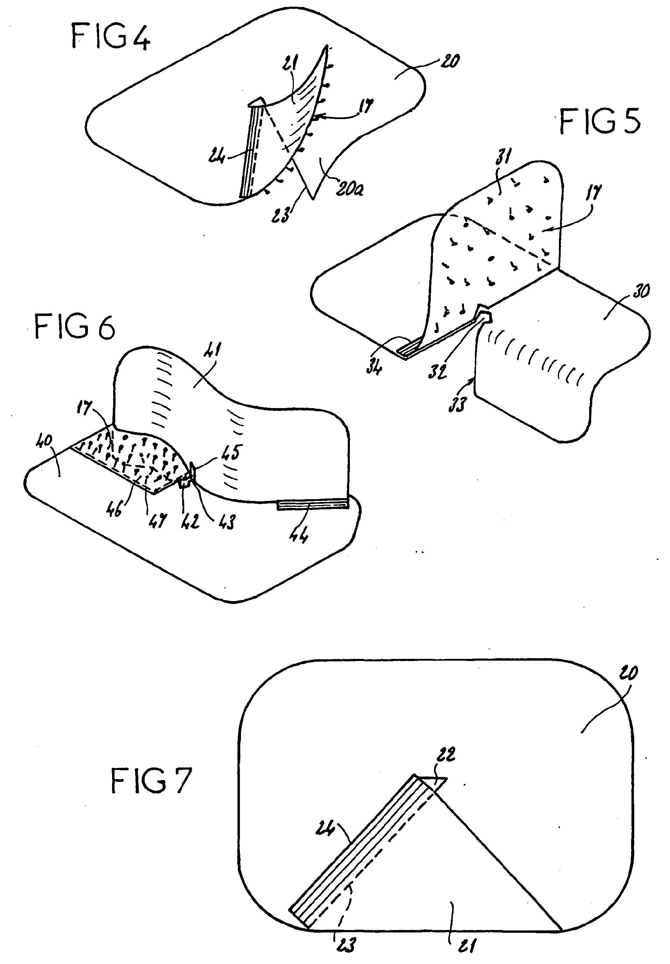

L'implant, représenté aux figures 4 et 7, destiné au renforcement de la région inguinale par voie postérieure, est composé d'un élément 20 réalisé en tricot prothétique 18, et en conséquence, ajouré et poreux, et d'un élément agrippant 21 réalisé dans le tricot agrippant 14 selon l'invention. L'élément 20 a la forme générale d'un rectangle à bords arrondis et comporte une ouverture centrale 22, ici de forme triangulaire, dans laquelle débouche une fente 23 délimitant le volet postérieur 20a de l'implant. Le bord de la fente opposé au volet 20a supporte la zone de liaison 24, par soudure ou couture, de l'élément 21, qui présente une forme triangulaire formant un volet antérieur se superposant en totalité sur le volet postérieur, ou autre élément non agrippant 20a. La figure 4 montre que les poils à picot 17 sont dirigés de manière à saillir en direction du volet 20a.

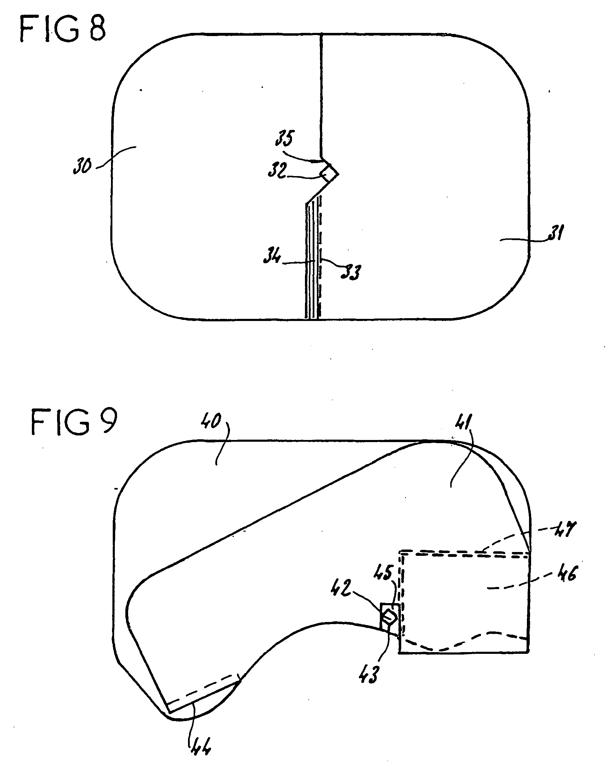

Dans l'implant de renfort représenté aux figures 5 et 8, également destiné au renforcement de la région inguinale par voie postérieure, l'élément 30 en tissu prothétique standard 18 a la forme d'un rectangle, à bords arrondis, et comporte une ouverture centrale 32, de forme carrée, communiquant par une fente 33 avec l'extérieur. L'élément agrippant 31 s'étend sur la moitié de l'élément 30 et est fixé sur celui-ci au niveau d'une zone de liaison 34, par soudure ou couture. Pour ne pas occulter l'ouverture 32 lorsqu'il est rabattu sur l'élément 30, l'élément 31 comporte, au voisinage de cette ouverture, une encoche 35. La figure 5 montre que, comme dans la forme d'exécution précédente, les poils à picot 17 de l'élément 31 sont tournés en direction du volet 30 sur lequel ils doivent être rabattus.

On notera que, dans ces implants, les éléments 21 et 31, réalisés en tricot agrippant 14 et destinés à être rabattus sur l'élément, respectivement 20 et 30 en tricot non agrippant 18, sont conformés pour s'inscrire, après leur rabattement, dans la forme de l'élément 20 et 30, de manière qu'en aucun cas les poils à picot 17 puissent venir en contact avec les tissus biologiques environnants.

L'implant représenté aux figures 6 et 9, ayant la même utilisation que les implants précédents mais conçu pour une technique chirurgicale différente, est composé de deux éléments 40 et 41 en tissu prothétique non agrippant 18. Ces deux éléments ont des conformations définies en fonction de l'anatomie et sont liés l'un à l'autre par une couture 44. L'élément 40 comporte une ouverture 42, de forme carrée et prolongée par une fente 43, et l'élément 41 comporte, dans sa partie devant venir sur l'ouverture 42 et la fente 43, une encoche 45. La liaison des deux éléments 40 et 41, après pose, est assurée par une pièce 46 de tricot agrippant 14 liée par une couture 47 à l'élément 40. Cette pièce est disposée avec les poils à picot 17 saillant de la face antérieure de l'élément 40.

Dans un implant selon la présente invention, l'élément agrippant peut être intégré dans ou rapporté sur la structure textile dudit implant.

Tous ces implants, destinés à être posés par voie postérieure, passent très aisément à travers un trocart, en étant replié avec les poils à picot saillant vers l'extérieur. Une fois mis en place, les éléments de l'implant sont dépliés pour ajuster la position de celui-ci, mais aussi pour faire passer un organe, par exemple le conduit spermatique, par l'ouverture 22, 32 ou 42. Lorsque l'implant est parfaitement étalé et bien positionné, le rabattement de l'élément 21, 31 ou 41 sur l'autre élément assure, par le caractère auto-agrippant des poils à picot 17, l'auto-accrochage de ces éléments, et le renfort de la région inguinale la plus fragile, à savoir le fascia transversalis.

En cas de besoin, le chirurgien peut séparer les volets agrippés, puis les reverrouiller.

Par sa réalisation satisfaisant aux contraintes des renforts prothétiques le tricot agrippant est sans conséquence sur le réhabitation tissulaire. Le monofil constituant les poils à picot est en matériau biorésorbable et les poils à picot disparaissent quand la réhabitation tissulaire est suffisante pour assurer la liaison des éléments de l'implant.

Il va de soi que l'invention n'est pas limitée à la forme de réalisation décrite ci-dessus à titre d'exemple mais qu'elle en embrasse au contraire toutes les variantes de réalisation.

Ainsi, le tricot 14 peut être résorbable, ou être en partie résorbable.

Les différents matériaux qu'il est possible d'utiliser pour les parties de l'implant sont indiqués ci-après :
- fil de formation des boucles donnant naissance aux poils à picots : polymère d'acide lactique monofilament ;
- chaîne du tricot : polypropylène monofilament, polypropylène multifilaments, polymère d'acide lactique multifilaments ou polyéthylène théréphtalate multifilaments ;
- trame du tricot : polypropylène monofilament, polypropylène multifilaments, polymère d'acide lactique multifilaments ou polyéthylène théréphtalate multifilaments ;
- fil formant les bouclettes d'ancrage des poils à picots et/ou constituant le tricot prothétique sur lequel est appliqué le tricot 14 : polypropylène multifilaments, polymère d'acide lactique multifilaments ou polyéthylène théréphtalate multifilaments.

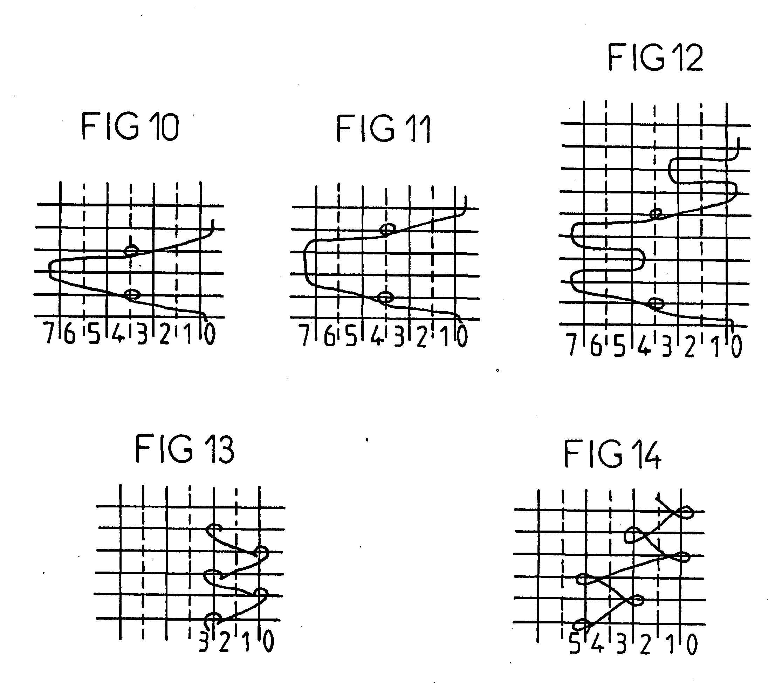

D'autres types d'armures que celles décrites plus haut peuvent être envisagées. Ainsi, la barre arrière peut se déplacer selon l'une des armures indiquées ci-dessous, en fonction de la densité de poils à picots recherchée :
- 00 / 43 / 77 / 34 / 00 (cf. figure 10) ;
- 00 / 43 / 77 / 77 / 34 / 00 (cf. figure 11);
- 00 / 43 / 77 / 44 / 77 / 34 / 00 / 33 / 00 (cf. figure 12).

La barre intermédiaire peut être enfilée, une passette pleine, une passette vide, travaillant en armure chaînette 10 / 01 ou 01 / 10.

La barre avant peut être enfilée pleine ou à disposition avec un fil ou avec deux fils de matériaux différents, en provenance d'un ou de deux rouleaux, et réaliser l'une ou l'autre des armures suivantes :
- 32 / 01 (cf. figure 13) ;
- 45 / 32 / 45 / 01 / 23 / 10 (cf. figure 14).

Le tricot prothétique ainsi obtenu a pour avantage d'être auto-accrochant ou auto-aggripant par retournement. Les poils à picot peuvent venir s'accrocher sur la face du tricot dépourvue de poils à picot, par retournement ou superposition envers sur endroit. Ce même tricot prothétique peut être utilisé pour un accrochage directement dans les tissus de la paroi traitée, parce que les poils à picots sont réalisés en fil résorbable.

## Revendications

1. Tricot prothétique à usage médical ou chirurgical dont la structure est réalisée en fil monofilament et multifilament, biocompatible et partiellement biorésorbable, **caractérisé en ce qu'**il comprend au moins trois nappes, dont une nappe en monofil (10) thermofusible formant, sur une face du tricot, des poils à picot (17) saillant perpendiculairement à ladite nappe, c'est-à-dire des poils (17) présentant chacun un corps sensiblement rectiligne et, du côté de l'extrémité libre de ce corps, une tête (17a) de largeur supérieure à celle de ce corps, ladite structure comportant sur ses deux faces, dont celle comprenant les poils à picot (17), des pores ouverts, le monofil formant les poils à picot étant en matériau bio-résorbabte thermofusible, choisi dans le groupe consistant en les polymères de p-dioxanone, les polyglycolides, les polyorthoesters, les polymères de triméthylène carbonate, les stéréocopolymères de l'acide L et D lactique, les homopolymères de l'acide L lactique, les copolymères de l'acide lactique et d'un comonomère compatible, tels que les dérivés d'alpha-hydroxy acides.

2. Tricot selon la revendication 1, **caractérisé en ce que** la longueur des poils à picot (17) est définie pour pénétrer et s'accrocher dans la structure textile du tricot, de manière limitée, sans en dépasser, par exemple par retournement d'une face agrippante dudit tricot, comportant lesdits poils à picot contre une face non agrippante, ne comportant pas lesdits poils, du même tricot ou d'un tricot différent.

3. Tricot selon la revendication 1 ou 2, **caractérisé en ce que** le monofil (10) formant les poils à picot (17) a un diamètre supérieur à 0,10 mm.

4. Tricot selon l'une quelconque des revendications 1 à 3, **caractérisé en ce que** chaque poil à picot (17) a une longueur comprise entre 1 et 2 mm.

5. Tricot selon l'une des revendications 1 à 4, **caractérisé en ce que** la densité des poils à picot (17) est comprise entre 50 et 90 poils au cm².

6. Tricot selon l'une des revendications 1 à 5, **caractérisé en ce que** les pores ouverts ont un diamètre compris entre 1 et 3 mm.

7. Utilisation d'un tricot selon l'une quelconque des revendications 1 à 6, pour l'obtention d'un objet prothétique, à usage médical ou chirurgical.

8. Implant prothétique de renfort tissulaire, **caractérisé en ce qu'**il est constitué au moins en partie par un tricot selon l'une quelconque des revendications 1 à 6.

## Claims

1. A prosthetic knit for medical or surgical use whose structure is made up of monofilament and multifilament yarn which is biocompatible and partially bioabsorbable, wherein it comprises at least three sheets, among which a thermofusible monofilament sheet (10) forming, on one face of the knit, spiked naps (17) which protrude perpendicularly with respect to said sheet, that is to say naps (17) each having a substantially rectlinear body and, at the free end of this body, a head (17a) of greater width than that of this body, said structure defining on its two faces, including the one with the spiked naps (17), open pores, the monofilament forming the spiked naps being made of a thermofusible bioabsorbable material chosen from the group consisting of the polymers of p-dioxanone, polyglycolides, polyorthoesters, polymers of trimethylene carbonate, stereocopolymers of L-lactic acid and D-lactic acid, homopolymers of L-lactic acid, copolymers of lactic acid and a compatible comonomer, such as derivatives of alpha-hydroxy acids.

2. The knit as claimed in claim 1, wherein the length of the spiked naps (17) is defined so as to penetrate and fasten in the textile structure of the knit in a limited manner, without emerging from it, for example when a grip face of said knit comprising said spiked naps is turned back against a non-grip face, not comprising said naps, of the same knit or of a different knit.

3. The knit as claimed in claim 1 or claim 2, wherein the monofilament (10) forming the spiked naps (17) has a diameter of over 0.10 mm.

4. The knit as claimed in one of claims 1 through 3, wherein each spiked nap (17) has a length of between 1 and 2 mm.

5. The knit as claimed in one of claims 1 through 4, wherein the density of the spiked naps (17) is between 50 and 90 naps per cm².

6. The knit as claimed in one of claims 1 through 5, wherein the open pores have a diameter of between 1 and 3 mm.

7. The use of the knit as claimed in any one of claims 1 through 6, for obtaining a prosthetic article for medical or surgical use.

8. A prosthetic implant for tissue reinforcement, wherein it is formed at least in part by the knit as claimed in any one of claims 1 through 6.

## Patentansprüche

1. Prothetisches Gewirk für medizinischen oder chirurgischen Gebrauch, dessen Struktur aus biokompatiblem und teilweise bioresorbierbarem Monofilament- und Multifilamentfaden gefertigt ist, **dadurch gekennzeichnet, dass** es zumindest drei Fadenlagen aufweist, von denen eine Fadenlage aus warmschmelzbarem Monofaden (10) auf einer Seite des Gewirkes Zähnchenhaare (17) bildet, die senkrecht zu der Fadenlage vorspringen, d.h. Haare (17), die jeweils einen etwa geradlinigen Körper und seitens des freien Endes dieses Körpers einen Kopf (17a) von größerer Breite als derjenigen des Körpers aufweisen, wobei die Struktur auf ihren beiden Seiten, unter ihnen diejenige, die die Zähnchenhaare (17) aufweist, offene Poren aufweist, wobei der Monofaden, der die Zähnchenhaare bildet, aus einem warmschmelzbaren bioresorbierbaren Material ist, das aus der Gruppe ausgewählt ist, die aus den p-Dioxanonpolymeren, den Polyglycoliden, den Polyorthoestern, den Trimethylencarbonatpolymeren, den Stereocopolymeren von der L- und D-Milchsäure, den Homopolymeren von der L-Milchsäure, den Copolymeren von der Milchsäure und einem kompatiblen Comonomer, so wie die Derivate von Alpha-Hydroxysäuren, besteht.

2. Gewirk nach Anspruch 1, **dadurch gekennzeichnet, dass** die Länge der Zähnchenhaare (17) dazu ausgelegt ist, damit sie in die textile Struktur des Gewirkes begrenzt eindringen, ohne durch es hindurchzugehen, und sich darin festhalten können, beispielsweise durch Umlegen einer greifenden Seite des Gewirkes, die die Zähnchenhaare aufweist, gegen eine nicht greifende Seite desselben Gewirkes oder eines anderen Gewirkes, die nicht die Haare aufweist.

3. Gewirk nach Anspruch 1 oder 2, **dadurch gekennzeichnet, dass** der Monofaden (10), der die Zähnchenhaare (17) bildet, einen Durchmesser von größer als 0,10 mm aufweist.

4. Gewirk nach einem der Ansprüche 1 bis 3, **dadurch gekennzeichnet, dass** jedes Zähnchenhaar (17) eine Länge im Bereich von 1 bis 2 mm aufweist.

5. Gewirk nach einem der Ansprüche 1 bis 4, **dadurch gekennzeichnet, dass** die Dichte der Zähnchenhaare (17) im Bereich von 50 bis 90 Haare pro cm² liegt.

6. Gewirk nach einem der Ansprüche 1 bis 5, **dadurch gekennzeichnet, dass** die offenen Poren einen Durchmesser im Bereich von 1 bis 3 mm aufweisen.

7. Verwendung eines Gewirkes nach einem der Ansprüche 1 bis 6 zum Herstellen eines prothetischen Erzeugnisses für medizinischen oder chirurgischen Gebrauch.

8. Prothetisches Implantat zur Gewebsverstärkung, **dadurch gekennzeichnet, dass** es zumindest teilweise aus einem Gewirk nach einem der Ansprüche 1 bis 6 gebildet ist.
